# EUROPEAN PATENT SPECIFICATION

(11) **EP 0 601 340 B1**
(45) Date of publication and mention of the grant of the patent: **12.07.2000**
(21) Application number: 93118004.6
(22) Date of filing: 05.11.1993
(51) Int. Cl.: A61N 1/05, A61N 1/39

(54) **Electrode system for a defibrillator**
Elektrodensystem für ein Defibrillationsgerät
Système d'électrodes pour un défibrillateur

(30) Priority: 11.12.1992 SE 9203735
(43) Date of publication of application: 15.06.1994
(73) Proprietor: Pacesetter AB, 175 84 Järfälla (SE)
(72) Inventor: Hirschberg, Jakub, S-183 44 Täby (SE); Wolf, Jens, S-121 45 Johanneshov (SE); Bowald, Staffan, S-740 10 Almunge (SE)

(56) References cited:
- WO-A-92/11898
- WO-A-92/18198
- US-A- 4 503 569
- US-A- 4 727 877
- US-A- 4 886 062
- US-A- 5 099 838
- US-A- 5 163 427
- US-A- 5 165 403

## Description

This invention relates generally to an electrode system intended for proximal connection to a cardioverter/defibrillator and for distal placement in the heart region in order to deliver electrical energy to the heart from the cardioverter/defibrillator to terminate an arrhythmia in the heart. More specifically, the invention relates to a system with at least two electrodes as set forth in the beginning of patent claim 1.

In defibrillation/cardioversion (cardioversion in this context refers to lower energy defibrillation; the collective designation defibrillation will be used hereinafter) with the aid of e . g. a three-electrode system with two intravascular electrodes, one of these intravascular electrodes is normally placed in the right ventricle and another in either the superior vena cava or, less commonly, the inferior vena cava or, possibly, the coronary sinus and its tributary along the base of the heart. In order to achieve high utilization of the energy stored in the defibrillator and good distribution of current in the heart, while simultaneously obviating the need for major surgery (thoracostomy), the third electrode is devised as a subcutaneous, large-area electrode, i. e. a patch electrode. The patch electrode is usually placed in the vicinity of the left ventricle, between the patient's ribs and skin.

Electrode systems with the described placements are shown, among others, in US-A-4,708,145. In addition to the intravascular electrodes shown there with a common electrode cable for a plurality of electrodes in the right ventricle and superior vena cava, separate electrode cables for different electrodes and a separate sensor electrode to detect cardiac events are also possible.

This is shown in US-A-4,727,877, EP A-O 373 953 and US-A-5,044,375 respectively. The use of systems with only two (non-epicardiac) electrodes is also known. One example of this is shown in US-A-3,942,536, in which the electrodes, located along the same electrode cable, are placed in the superior vena cava and right ventricle respectively.

Another example of prior art is disclosed in WO 92/11898 which shows unspecified electrodes devised for anchoring within blood vessels. For example, an electrode is shown anchored in the coronary sinus. A further example of a related electrode system is disclosed in WO 92/18198, which shows a combination of a defibrillation electrode placed in the coronary sinus and sensing electrodes placed in the great vein.

When the heart's coronary vessels on the venous side are put to use as the site of at least one of the defibrillation electrodes in prior art systems, the vessels used have been the coronary sinus and its tributary, the great cardiac vein, along the base of the heart or, to put it another way, along the heart's valve plane.

The present invention is based on the inventors' understanding that the venous side of the coronary vessels running between the base and apex of the heart, generally referred to as the heart's peripheral veins, such as the great, middle and small cardiac veins, offers opportunities for placing at least one defibrillation electrode, this electrode, interacting with the other electrodes in the system, having been shown to produce high utilization of the energy stored in the defibrillator and favorable distribution of current in the heart. In view of the described prior art, the favorable effect of this placement is surprising, since e.g. the above-mentioned EP-A 0 373 953 emphasizes the importance of restricting electrode placement to coronary vessels along the base of the heart (col 6, lines 24-33).

The object of the invention is to achieve an electrode system in which at least one electrode is sited in a coronary vessel and in which high utilization of the energy stored in the defibrillator is combined with a favorable distribution of current in the heart.

This object is achieved with an electrode system of the above-described kind with the features specified in patent claim 1.

According to the invention, an electrode system is thereby attained in which two of the electrodes are devised for intravenous placement in the coronary sinus and the heart's peripheral veins.

Said at least two venous electrodes are arranged along a common electrode cable and are equipped with means for affixing them to the inner venous wall. The fixation means can have the shape of a hollow, resilient cylinder which achieves fixation by means of radial expansion. The hollow cylindrical shape of the electrode enables blood to flow unimpeded through the electrode. Other advantageous embodiments are noted in the sub-claims.

The electrode system according to the invention is presented below in greater detail, referring to an attached drawing in which
FIG. 1 schematically depicts a heart in cross-section (frontal plane) with the electrode system connected to the heart according to a first embodiment of the invention;
FIGS. 2-4 schematically depict a heart in cross-section (frontal plane) with the electrode system connected to the heart according to other embodiments of the invention.

Identical reference designations designate identical or similar parts in all the FIGURES.

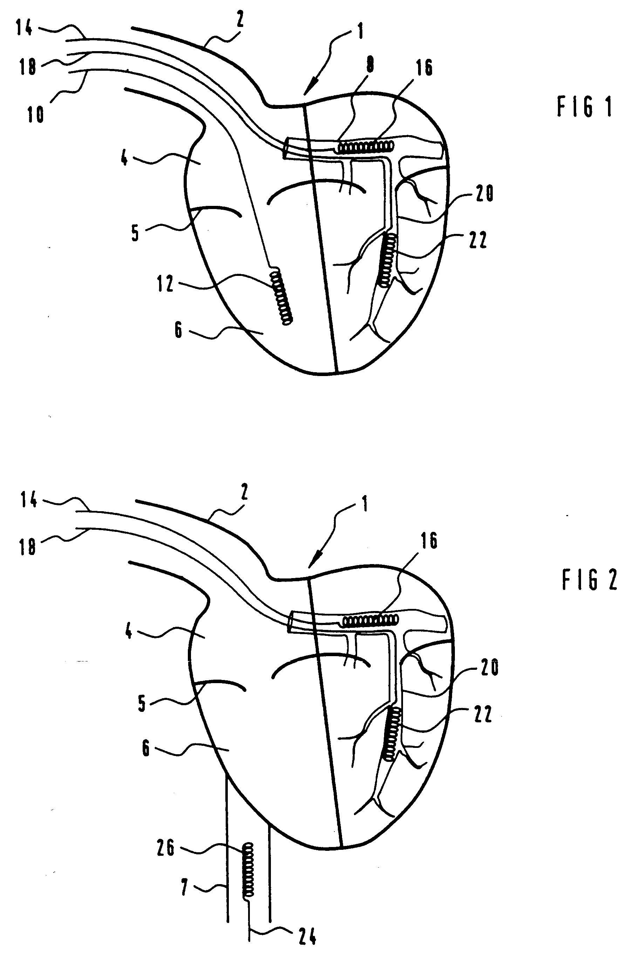

FIG. 1 shows a cross-section of a heart 1 and a plurality of blood vessels important to the invention.

A first electrode cable 10, introduced via the superior vena cava 2, passes through the right atrium 4 and valve plane 5 and exits into the right ventricle 6. At or near its distal end, the cable 10 is provided with an electrode 12 which can be anchored in the right ventricle. A second electrode cable 14, also introduced via the superior vena cava 2, passes through the right atrium 4 but, in contrast to the cable 10, in such a way that it exits into the coronary sinus 8. At its distal end, the cable 14 is provided with an electrode 16 which can be anchored in the coronary sinus. A third electrode cable 18, also introduced via the superior vena cava 2, passes through the right atrium 4 and coronary sinus 8 and possibly its tributary so the cable exits into a peripheral vein 20, shown in FIG. 1, of the heart 1. At its distal end, the cable 18 is provided with an electrode 22 which can be anchored in the peripheral vein 20. The electrodes 12, 16, 22 are connectable to the defibrillator (not shown) via existing electrical conductors in the respective cables 10, 14, 18. The two cables 14, 18 are a common cable.

The electrode system according to FIG. 2 differs from the system shown in FIG. 1 because the electrode 12 placed in the right ventricle 6 has been replaced with the electrode 26, placed in the inferior vena cava 7, which is connectable to the defibrillator via an existing conductor in the electrode cable 24.

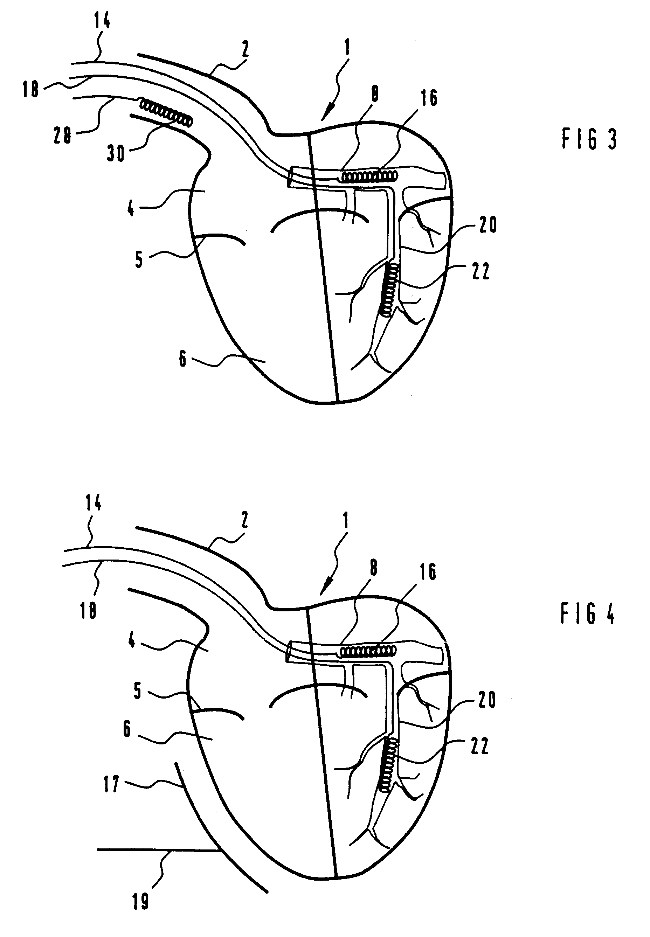

The electrode system according to FIGS. 3 and 4 differs from the system in FIG. 1 because the electrode 12 placed in the right ventricle 6 is replaced by the electrode 30 (FIG. 3) placed in the superior vena cava 2 or with a subcutaneous patch electrode 17 (FIG. 4) placed near the right ventricle 6. The electrodes 30 and 17 are connectable to the defibrillator via existing conductors in the electrode cable 28 and 19 respectively. "Subcutaneous" placement here refers to placement between the ribs and skin. The defibrillator's enclosure can alternately be used as a patch electrode.

Connection of the electrodes according to FIGS. 1-4 to the defibrillator and to one another can be achieved in different ways. For example, the electrodes 16, 22 shown in FIG. 1 can be inter-connected in such a way that the defibrillation pulse passes between these electrodes, on the one hand, and the electrode 12 in the right ventricle 6, on the other hand.

However, the electrodes 12, 16, 22 can be inter-connected so the defibrillation pulse passes between one of these electrodes, on the one hand, and the other two electrodes, on the other hand. Alternately, each of the electrodes 12, 16, 22 can be given a different voltage in defibrillation or, as an additional alternative, emit defibrillation pulses sequentially in pairs. Monophasic, biphasic or multiphasic defibrillation pulses can be used.

The cables 14, 18 for the two electrodes 16, 22 shown in FIG. 1, are a common electrode cable for both the electrodes 16, 22. The inferior vena cava 7 can be used as an alternative to the described routing of intravascular electrodes through the superior vena cava 2. The intravascular electrodes introduced via the former route can also share an electrode cable or have separate electrode cables. In the configuration shown in FIG. 3, the electrodes 16, 22 30 can have e.g. a single, common catheter.

All configurations, with the exception of the subcutaneous patch electrode 17, using the same implantation route for a plurality of electrodes, could also obviously be arranged on a common electrode cable.

The described electrode configurations can also be supplemented with a separate sensor electrode for detecting cardiac events and/or a sensor for sensing different parameters related to e.g. cardiac hemodynamics. The sensor electrode's electrode cable could also include a stimulation electrode for a pacing function.

The intravascular electrodes 16, 22, 26, 30 are kept in place in the respective vein by a construction making them radially expandable, forming in the expanded position at least the contours of a hollow cylinder. Defibrillation electrodes of this type are described in detail in the applicants' simultaneously filed European patent application entitled "Defibrillation Electrode", their reference GR 92 P 7364, Appl No 93118003.8, this document serving as a part of the present application so as to avoid repetition here. Thus, the intravascular electrodes 16, 22, 26, 30 are kept in place in the respective vein by being devised as helices whose bias tension, transverse to the longitudinal axis of the helix, exerts pressure on the venous wall. To avoid repetition, only helical fixation of the electrode 26 in the inferior vena cava 7 will be described below, but the description obviously covers the other intravascular electrodes. In its pre-shaped state, the helical electrode 26 can be envisaged as being helically wound around an imaginary cylinder whose external diameter is somewhat larger that the inner diameter of the inferior vena cava 7. The helix can be made of an electrically conductive, biocompatible material. The electrode 26 is connected to the electrode cable 24 in such a way that they jointly form a continuous unit. A centrally located longitudinal channel, into which a stylet can be introduced, runs through the electrode 26 and the electrode cable 24. During implantation, the electrode 26 is straightened out with the aid of the stylet. The electrode 26, whose diameter at implantation is therefore smaller than the diameter of the blood vessels it is to traverse, can then be introduced into the inferior vena cava 7. When the implanting physician reaches an appropriate site for the electrode 26 in the inferior vena cava, the stylet is withdrawn, and the electrode 26 then reassumes its preshaped, helical configuration. The pressure of the helix against the venous wall keeps the helix at the desired site. In the affixed position, the electrode 36 forms a relatively large electrode surface against the vascular wall. At the same time, the helical electrode 26 has the advantage of allowing blood in the vessel to flow unimpeded through the interior of the helix. The risk of clot formation is thereby minimized. In addition, the electrode 26 can be easily repositioned if the stylet, reinserted into the central channel, is used to straighten out the electrode 26. Thus, the implanted physician can readily inferior vena cava 7, the stylet is withdrawn find a site for the electrode 26 which, with the other electrodes, provides favorable distribution of current in heart tissue.

In implantation, the physician can use an introductory catheter instead of a stylet. The electrode cable 24 with the straightened electrode 26 is inserted in the introductory catheter, the latter being sufficiently stiff to keep the electrode 26 straightened out during implantation. When the electrode 26 has been advanced to the desired position in the inferior vena cava 7, the introductory catheter can be withdrawn so the electrode reassumes its precoiled configuration.

In instances in which the electrodes, e.g. electrodes 16, 22, are arranged on a common electrode cable, implantation can be performed with the aid of a single stylet running through a central channel through all the electrodes and the electrode cable. Both electrodes are then straightened out after implantation.

When the electrode 22 reaches the desired position, the stylet is withdrawn just enough to allow the electrode 22 to reassume its helical configuration, the electrode 16 thereafter being positioned at the desired site in the coronary sinus 8. When the electrode 16 has been properly positioned, the stylet is withdrawn completely so the electrode 16 reassumes its preshaped configuration. An introductory catheter could also be used here instead of a stylet.

The electrode 16 intended for placement in the coronary sinus 8 and its tributary along the base of the heart could consist of e.g. two sub-electrodes placed in the coronary sinus 8. The two sub-electrodes can be arranged on a common extension of e.g. the electrode cable 14. The two sub-electrodes are placed in the coronary sinus 8, additionally improved distribution of current in the heart is attained.

Implantation here is carried out in the above-described way. More than two sub-electrodes could be used. A system employing subelectrodes could also be employed for the electrode 22 placeable in a peripheral vein 20.

## Claims

1. An electrode system for a defibrillator with at least two intravenously placeable electrodes, wherein at least two of the intravenously placeable electrodes are separately arranged on a common electrode cable, characterized in that a first electrode (22) on said common electrode cable is devised for intravenous placement in one of the heart's peripheral veins (20) and in that a second electrode (16) on said common electrode cable is devised for intravenous placement in the coronary sinus (8), the intravenous electrodes in the electrode system having means for affixing the electrodes to the inner wall of the vein in which they are located, the means for fixation being achieved when the electrode in the affixed position has a hollow, cylindrical shape whose radius exceeds the radius of the vein enough to affix the electrode by pressure to the inner wall of the vein, said first and second electrode (16, 20) being pre-shaped so as to have a diameter in the pre-shaped state that is somewhat larger than the inner diameter of the respective vein in which said electrodes are to be placed.

2. The electrode system of claim 1, characterized in that the system comprises a third electrode.

3. The electrode system of claim 2, characterized in that the third electrode (12) is devised for placement in the right ventricle (6).

4. The electrode system of claim 2, characterized in that the third electrode (26) is devised for intravenous placement in the inferior vena cava (7).

5. The electrode system of claim 2, characterized in that the third electrode (30) is devised for intravenous placement in the superior vena cava (2).

6. The electrode system of claim 2, characterized in that the third electrode consists of a subcutaneous patch electrode (17) placeable in the vicinity of the right ventricle (6).

7. The electrode system of any of the preceding claims, characterized in that the intravenously placeable electrode consists of a helix whose helical turns comprise at least a part of the cylinder's mantle.

8. The electrode system of claim 6, characterized in that the patch electrode consists of at least a part of the defibrillator enclosure.

9. The electrode system of any of the preceding claims, characterized in that the electrode intended for placement in the coronary sinus and/or in a peripheral cardiac vein consists of at least two separate sub-electrodes, the most distal of the at least two sub-electrodes connected to the proximal sub-electrode by a common electrode cable.

## Patentansprüche

1. Ein Elektrodensystem für einen Defibrillator mit mindestens zwei intravenös plazierbaren Elektroden, wobei zumindest zwei der intravenös plazierbaren Elektroden separat auf einem gemeinsamen Elektrodenkabel angeordnet sind, **dadurch gekennzeichnet,** daß eine erste Elektrode (22) auf dem gemeinsamen Elektrodenkabel zur intravenösen Plazierung in einer der peripheren Venen (20) des Herzens ausgebildet ist und daß eine zweite Elektrode (16) auf dem Elektrodenkabel zur intravenösen Plazierung in dem Sinus coronarius (8) ausgebildet ist, wobei die intravenösen Elektroden in dem Elektrodensystem Mittel zur Befestigung der Elektroden an der Innenwand der Vene, in der sie angeordnet sind, aufweisen, wobei die Befestigungsmittel erzielt werden, wenn die Elektrode in der fixierten Position eine hole, zylindrische Form hat, deren Radius den Radius der Vene genügend übersteigt, um die Elektrode durch Druck auf die Innenwand der Vene zu befestigen, wobei die erste und zweite Elektrode (16, 20) so vorgeformt sind, daß sie in dem vorgeformten Stadium einen Durchmesser haben, der etwas größer ist als der Innendurchmesser der entsprechenden Vene, in der die Elektroden plaziert werden sollen.

2. Das Elektrodensystem nach Anspruch 1, **dadurch gekennzeichnet,** daß das System eine dritte Elektrode aufweist.

3. Das Elektrodensystem nach Anspruch 2, **dadurch gekennzeichnet,** daß die dritte Elektrode (12) zur Plazierung in dem rechten Ventrikel (6) ausgebildet ist.

4. Das Elektrodensystem nach Anspruch 2, **dadurch gekennzeichnet,** daß die dritte Elektrode (26) zur intravenösen Plazierung in der Vena cava inferior (7) ausgebildet ist.

5. Das Elektrodensystem nach Anspruch 2, **dadurch gekennzeichnet,** daß die dritte Elektrode (30) zur intravenösen Plazierung in der Vena cava superior (2) ausgebildet ist.

6. Das Elektrodensystem nach Anspruch 2, **dadurch gekennzeichnet,** daß die dritte Elektrode aus einer in der Nähe des rechten Ventrikels (6) plazierbaren, subkutanen Patchelektrode (17) besteht.

7. Das Elektrodensystem nach einem der vorhergehenden Ansprüche, **dadurch gekennzeichnet,** daß die intravenös plazierbare Elektrode aus einer Spirale besteht, deren Spiralwindungen zumindest einen Teil des Zylindermantels aufweisen.

8. Das Elektrodensystem nach Anspruch 6, **dadurch gekennzeichnet,** daß die Patchelektrode aus zumindest einem Teil des Defibrillatorgehäuses besteht.

9. Das Elektrodensystem nach einem der vorhergehenden Ansprüche, **dadurch gekennzeichnet,** daß die für die Plazierung in dem Sinus coronarius und/oder in einer peripheren Herzvene vorgesehene Elektrode aus zumindest zwei separaten Unterelektroden besteht, wobei die distalste der zumindest zwei Unterelektroden über ein gemeinsames Elektrodenkabel mit der proximalen Unterelektrode verbunden ist.

## Revendications

1. Système d'électrodes pour un défibrillateur, comprenant au moins deux électrodes pouvant être placées dans les veines, dans lequel au moins deux électrodes pouvant être placées dans les veines sont disposées séparément sur un câble d'électrode commun, caractérisé en ce qu'une première électrode (22) disposée sur le câble d'électrode commun est agencée pour être mise dans l'une des veines périphériques du coeur (20), et en ce qu'une seconde électrode (16) disposée sur le câble d'électrode commun est agencée pour être mise dans le sinus coronarien (8), les électrodes intraveineuses du système d'électrodes ayant des moyens de fixation des électrodes à la paroi interne de la veine dans laquelle elles se trouvent, ces moyens de fixation étant mis en oeuvre lorsque l'électrode en position de fixation a une forme cylindrique creuse dont le rayon est supérieur au rayon de la veine d'une manière suffisante pour fixer l'électrode par pression à la paroi interne de la veine, les première et deuxième électrodes (16, 20) étant préformées de telle sorte qu'elles aient, à l'état préformé, un diamètre un peu plus grand que le diamètre interne de la veine respective dans laquelle les électrodes doivent être mises.

2. Système d'électrodes suivant la revendication 1, caractérisé en ce que le système comprend une troisième électrode.

3. Système d'électrodes suivant la revendication 2, caractérisé en ce que la troisième électrode (12) est agencée pour être mise dans le ventricule droit (6).

4. Système d'électrodes suivant la revendication 2, caractérisé en ce que la troisième électrode (26) est agencée pour être mise dans la veine cave inférieure (7).

5. Système d'électrodes suivant la revendication 2, caractérisé en ce que la troisième électrode (30) est agencée pour être mise dans la veine cave inférieure (2).

6. Système d'électrodes suivant la revendication 2, caractérisé en ce que la troisième électrode est constituée d'une électrode sous-cutanée en forme de pastille (17) pouvant être disposée à proximité du ventricule droit (6).

7. Système d'électrodes suivant l'une quelconque des revendications précédentes, caractérisé en ce que l'électrode pouvant être placée dans les veines est constituée d'une hélice dont les spires d'hélice représentent au moins une partie de l'enveloppe du cylindre.

8. Système d'électrodes suivant la revendication 6, caractérisé en ce que l'électrode sous forme de pastille est constituée d'au moins une partie du boîtier du défibrillateur.

9. Système d'électrodes suivant l'une quelconque des revendications précédentes, caractérisé en ce que l'électrode agencée pour être mise dans le sinus coronarien et/ou dans la veine périphérique du coeur consiste en au moins deux sous-électrodes distinctes, celle dont la position est la plus distale desdites au moins deux sous-électrodes étant connectée à la sous-électrode proximale par un câble d'électrode commun.
